# EUROPEAN PATENT SPECIFICATION

(11) **EP 4 154 879 B1**
(45) Date of publication and mention of the grant of the patent: **10.09.2025**
(21) Application number: 22167414.6
(22) Date of filing: 08.04.2022
(51) Int. Cl.: A61K 31/40, A61P 25/34

(54) **PHARMACEUTICAL COMPOSITION FOR PREVENTING OR TREATING NICOTINE WITHDRAWAL SYMPTOMS**
PHARMAZEUTISCHE ZUSAMMENSETZUNG ZUR VORBEUGUNG ODER BEHANDLUNG VON NIKOTINENTZUGSERSCHEINUNGEN
COMPOSITION PHARMACEUTIQUE POUR LA PRÉVENTION OU LE TRAITEMENT DES SYMPTÔMES DE SEVRAGE DE LA NICOTINE

(30) Priority: 28.09.2021 KR 20210127992
(43) Date of publication of application: 29.03.2023
(73) Proprietor: Korea Institute of Science and Technology, Seoul 02792 (KR)
(72) Inventor: IM, Heh-In, 02792 Seongbuk-gu (KR); KIM, Byung Sun, 02792 Seongbuk-gu (KR)
(74) Representative: advotec.

(56) References cited:
- WO-A1-2014/176460
- WO-A1-98/34615
- US-A- 4 555 397
- US-A1- 2012 302 942

## Description

### BACKGROUND OF THE INVENTION

### 1. Field of the Invention

The present invention relates to a pharmaceutical composition for preventing or treating nicotine withdrawal symptoms, and more specifically to a pharmaceutical composition for preventing or treating nicotine withdrawal symptoms including a muscarinic receptor antagonist as an active ingredient.

### 2. Description of the Related Art

Nicotine is a precursor of nitrosamines, including 4-(methylnitrosamino)-1-(3-pyridyl)-1-butanone (NKK), and is a potent environmental toxin. Nicotine is quickly absorbed anywhere in the body and reaches the brain within about 7 seconds, which explains its rapid action. The rapid action of nicotine positively reinforces smoking behavior. Nicotine reaching the brain acts on nicotinic acetylcholine receptors to enhance cholinergic neurotransmission in the brain. Particularly, nicotine acts directly on nicotinic acetylcholine receptors in the mesolimbic dopamine system to promote the secretion of dopamine from the ventral tegmental area to the nucleus accumbens and induce a sense of reward. Several experimental evidence reveals that nicotine has neurochemical and functional effects similar to other addictive drugs and increased dopaminergic neurotransmission plays a particularly important role in inducing symptoms of nicotine addiction or nicotine withdrawal.

Dopamine (4-(2-aminoethyl)benzene-1,2-diol) is an organic compound of the catecholamine family and is a neurotransmitter found in the central nervous system. Dopamine is mainly synthesized in neurons and adrenal medullary cells. In dopamine secreting neurons, L-DOPA is synthesized from tyrosine using the enzyme tyrosine hydroxylase (TH) and dopamine is produced from L-DOPA using the enzyme DOPA decarboxylase. Dopamine produced in neurons is transported to the nerve terminal by synaptic vesicles and is stored there. When dopamine is released by appropriate electrical stimulation, it binds to dopamine receptors present in postsynaptic neurons, resulting in signal transduction. Used dopamine is reuptaken into presynaptic neurons by the dopamine transporter (DAT) protein. Addictive substances such as nicotine increase dopamine synthesizing tyrosine hydroxylase (TH) and inhibits the action of the dopamine transporter (DAT) protein to increase synaptic dopamine concentration, leading to arousal.

Nicotine replacement therapies (NRTs) such as nicotine transdermal patches or nicotine-containing gums, and pharmacotherapy such as varenicline and bupropion have been developed to address nicotine withdrawal. They deliver a certain amount of nicotine to users through routes other than smoking to help them distract away from the desire to smoke but allow only a small amount of nicotine to penetrate the bloodstream, which increases the desire to smoke in many cases. Further, nicotine replacement therapies may cause side effects such as oral inflammation, gnathalgia, nausea, skin irritation, sleep disorder, and nervousness. Since nicotine withdrawal symptoms are accompanied by psychological dependence that cannot be treated with nicotine replacement therapy alone, they cannot be treated completely and have a high risk of recurrence.

Document WO 98/34615 A1 discloses the use of muscarinic antagonists (ipratropium and scopolamine) to alleviate withdrawal symptoms. The drugs were administered via the oral mucosa or sublingual. The document also mentions other muscarinic antagonists that can be sued singly or in combination, like a.o. homatropine, atropine, methscopolamine, ipratropium, benactyzine, benzotropine, trihexyphenidyl, biperiden and procyclidine.

Document US 2012/302942 A1 relates to a transdermal devices and methods for controllable dispensing of a.o. medication. Among the medications that can be used for this purpose, the said document mentions anticholinergic drugs such as atropine and scopolamine to help reduce nicotine withdrawal symptoms.

Document US 4 555 397 A relates to a method for anti-cholinergic blockage of withdrawal symptoms in smoking cessation. According to the said document, a subcutaneous injection of a solution comprising atropine, scopolamine and chlorpromazine alleviates withdrawal symptoms created by cessation of chronic nicotine use. Furthermore, as follow-up treatment, other anti-cholinergic drugs such as trihexyphenidyl hydrochloride, benztropine mesylate or scopolamine patches are mentioned to be taken by the patient.

Document WO 2014/176460 A1 relates to a method for lessening the symptoms of depressive disorders such as nicotine-induced disorders, including nicotine withdrawal syndrome. The method comprises the administration of cholinergic M1 receptor antagonist typically selected from the group consisting of aclidinium, amitriptyline, amoxapine, anisotropine methylbromide, aripiprazole, atropine, benzquinamide, biperiden, brompheniramine, buclizine, chlorprothixene, clidinium, clozapine, cocaine, cyclopentolate, cycrimine, cyproheptadine, darifenacin, desipramine, dicyclomine, diphenidol, disopyramide, doxepin, doxylamine, escitalopram, fesoterodine, flavoxate, flupentixol, homatropine methylbromide, hyoscyamine, imipramine, ipratropium bromide, maprotiline, mepenzolate, methantheline, methotrimeprazine, nicardipine, nortriptyline, olanzapine, oxybutynin, oxyphencyclimine, oxyphenonium, paroxetine, pirenzepine, procyclidine, promazine, promethazine, propantheline, propiomazine, quetiapine, scopolamine, solifenacin, tiotropium, tolterodine, tridihexethyl, triflupromazine, trihexyphenidyl, tropicamide, trospium, and ziprasidone. The treatment may be administered a.o. via oral route or parentally and the formulation may be a.o. in the form of an injection, infusion, transdermal patch.

Thus, there is an urgent need for a more fundamental therapeutic agent for nicotine withdrawal.

### Prior Art Documents

### Patent Documents

Patent Document 1. Korean Patent Publication No. 10-2020-0140539

### SUMMARY OF THE INVENTION

The present invention is defined by the appended claims and has been made in an effort to solve the above-described problems and an object of the present invention is to provide a pharmaceutical composition for use in preventing or treating nicotine withdrawal symptoms comprising a muscarinic receptor antagonist as an active ingredient; wherein the nicotine withdrawal symptoms are retropulsion and/or hand tremor; and wherein the muscarinic receptor antagonist is selected from the group consisting of procyclidine, dexetimide, methixene, propiverine, benztropine, propenamine, propantheline, quinidine, glycopyrronium, orphenadrine, chlorphenoxamine, butylscopolamine, revefenacin, otilonium, emepronium, bornaprine, propiomazine, promethazine, pipecuronium, pancuronium, terfenadine, chlorprothixene, brompheniramine, mivacurium, isopropamide, tramadol, umeclidinium, hexocyclium, dosulepin, imidafenacin, pizotifen, thonzylamine, gallamine triethiodide, rocuronium, doxacurium, metocurine, dimetindene, diphenhydramine, rapacuronium, lamotrigine, camylofin, difemerine, dihexyverine, mebeverine, piperidolate, rociverine, trimebutine, emetonium iodide, benzilone, bevonium, diphemanil, fenpiverinium, penthienate, pipenzolate, poldine, prifinium, tiemonium iodide, timepidium, phenglutarimide, oxitropium, tropatepine, mazaticol, etybenzatropine, etanautine, batefenterol, and mixtures thereof.

The muscarinic receptor antagonist may be an antagonist of muscarinic acetylcholine receptor M1, muscarinic acetylcholine receptor M2, muscarinic acetylcholine receptor M3, muscarinic acetylcholine receptor M4, muscarinic acetylcholine receptor M5, and combinations thereof.

The muscarinic receptor antagonist may be procyclidine.

The composition may be formulated into an oral or parenteral preparation, the oral preparation may be selected from the group consisting of suspension, solution for internal use, emulsion and syrups and the parenteral preparation may be an injectable, transrectal or transdermal preparation.

The use of the muscarinic receptor antagonist enables the prevention or treatment of nicotine withdrawal symptoms, said symptoms being retropulsion and/or hand tremor.

### BRIEF DESCRIPTION OF THE DRAWINGS

These and/or other aspects and advantages of the invention will become apparent and more readily appreciated from the following description of the embodiments, taken in conjunction with the accompanying drawings of which:
Fig. 1 is an open field test design for determining the efficacy of a muscarinic receptor antagonist in preventing or treating nicotine withdrawal;
Fig. 2 shows the moving distances of mice analyzed in an open field test for Groups 1 to 3;
Fig. 3 shows the degrees of retropulsion analyzed in an open field test for Groups 1 to 3;
Fig. 4 is a behavioral observation test design for determining the efficacy of a muscarinic receptor antagonist in preventing or treating nicotine withdrawal; and
Fig. 5 shows the degrees of paw tremors analyzed in a behavioral observation test for Groups 1 to 3.

### DETAILED DESCRIPTION OF THE INVENTION

Several aspects and various embodiments of the present invention will now be described in more detail.

An aspect of the present invention is related to a pharmaceutical composition for preventing or treating nicotine withdrawal symptoms including a muscarinic receptor antagonist as an active ingredient, wherein said symptoms are retropulsion and/or hand tremor.

The present inventors have found that administration of a muscarinic receptor antagonist ameliorates nicotine withdrawal symptoms induced by nicotine addiction. The present invention has been accomplished based on this finding.

Therefore, the present invention can provide a pharmaceutical composition for preventing or treating nicotine withdrawal symptoms including a muscarinic receptor antagonist as an active ingredient and a method for treating nicotine withdrawal symptoms including administering to an individual in need of treatment a composition including a muscarinic receptor antagonist as an active ingredient.

As used herein, the terms "preventing", "ameliorating", and "treating" mean to reverse or alleviate nicotine withdrawal symptoms caused after quitting nicotine or to inhibit, delay or prevent the progress of the symptoms. The term "treating" refers to an action of treatment in the meaning of "therapy".

The pharmaceutical composition of the present invention may be directly used to prevent or treat nicotine withdrawal symptoms or may be administered as a therapeutic supplement for nicotine withdrawal symptoms with other pharmacological ingredients. Accordingly, the term "treatment" or "therapeutic agent" as used herein is intended to include the meanings of "supplementary treatment" or "therapeutic supplements".

The use of nicotine increases the level of the neurotransmitter dopamine, which stimulates reward pathways that regulate feelings of pleasure and mediate the desire to consume nicotine.

As used herein, the term "nicotine addiction" refers collectively to poisoning caused when nicotine is administered or taken and is intended to include acute and chronic addiction. Chronic nicotine addiction is defined as a state in which a person has lost the power of self-control to continuously seek or take nicotine despite an emotional state such as physical or mental impairment. That is, chronic nicotine addiction refers to long-term exposure to nicotine.

The term "withdrawal" refers collectively to symptoms of various mental disorders caused as a result of craving for nicotine during the withdrawal period without taking nicotine in a state in which highly addictive nicotine is repeatedly administered and nicotine dependence is high, psychological symptoms such as irritability, anger, hostility, aggression, fatigue, and depression, and physical symptoms such as decreased activity and hand tremor.

In the Examples section that follows, a muscarinic receptor antagonist was demonstrated to exhibit efficacy in preventing or treating nicotine withdrawal symptoms due to its ability to control behaviors resulting from nicotine withdrawal symptoms.

It is known that five types of muscarinic receptors designated as M1 to M5 exist, each of which binds to acetylcholine secreted from the nervous system or muscarine present found in poisonous mushrooms. Among them, muscarinic receptors M1-M3 are distributed in the lung. It has been found that the M1-M3 receptors are related to the parasympathetic ganglion, the parasympathetic nerve, and the bronchial smooth muscle, respectively.

Recent studies have revealed that muscarinic receptor M2 is associated with asthma, but the exact roles of these receptors on nicotine withdrawal symptoms have not yet been fully elucidated.

As used herein, the term "antagonist" refers to a molecule that partially or completely inhibits the effects of other molecules such as receptors or intracellular mediators by any mechanism.

For the purposes of the present invention, the muscarinic receptor antagonist is an antagonist of muscarinic receptors, preferably muscarinic acetylcholine receptor M1, muscarinic acetylcholine receptor M2, muscarinic acetylcholine receptor M3, muscarinic acetylcholine receptor M4, muscarinic acetylcholine receptor M5, and combinations thereof. Preferably, the muscarinic receptor antagonist is an antagonist that has an antagonistic effect on muscarinic acetylcholine receptor M1, muscarinic acetylcholine receptor M2, muscarinic acetylcholine receptor M3, muscarinic acetylcholine receptor M4, and muscarinic acetylcholine receptor M5.

The muscarinic receptor antagonist is preferably a substance that can bind to a muscarinic receptor to directly, indirectly or substantially interfere with, reduce or inhibit the biological activity of the muscarinic receptor. The muscarinic receptor antagonist is selected from the group consisting of procyclidine, dexetimide, methixene, propiverine, benztropine, propenamine, propantheline, quinidine, glycopyrronium, orphenadrine, chlorphenoxamine, butylscopolamine, revefenacin, otilonium, emepronium, bornaprine, propiomazine, promethazine, pipecuronium, pancuronium, terfenadine, chlorprothixene, brompheniramine, mivacurium, isopropamide, tramadol, umeclidinium, hexocyclium, dosulepin, imidafenacin, pizotifen, thonzylamine, gallamine triethiodide, rocuronium, doxacurium, metocurine, dimetindene, diphenhydramine, rapacuronium, lamotrigine, camylofin, difemerine, dihexyverine, mebeverine, piperidolate, rociverine, trimebutine, emetonium iodide, benzilone, bevonium, diphemanil, fenpiverinium, penthienate, pipenzolate, poldine, prifinium, tiemonium iodide, timepidium, phenglutarimide, oxitropium, tropatepine, mazaticol, etybenzatropine, etanautine, batefenterol, and mixtures thereof. Procyclidine is most preferred.

The muscarinic receptor antagonist may be extracted from organisms. Alternatively, the muscarinic receptor antagonist may be chemically synthesized by a suitable method known in the art or may be commercially available.

The muscarinic receptor antagonist exhibits an inhibitory effect on nicotine withdrawal symptoms, selected from retropulsion and hand tremor, due to its ability to reduce nicotine-induced withdrawal symptoms to normal levels. Particularly, the muscarinic receptor antagonist can ameliorate or treat physical symptoms caused by nicotine withdrawal. In conclusion, the muscarinic receptor antagonist is very effective in preventing or treating nicotine withdrawal.

The muscarinic receptor antagonist may be used in the form of a pharmaceutically or sitologically acceptable salt with the same efficacy as the muscarinic receptor antagonist.

As used herein, "pharmaceutically or sitologically acceptable" means that the salt is not toxic to cells or humans exposed to the composition.

The salt may be used in the form of either a pharmaceutically or sitologically acceptable base or acid salt. The base salt may be either an organic or inorganic base salt and may be selected from the group consisting of sodium, potassium, calcium, lithium, magnesium, cesium, aminium, ammonium, triethyl, and pyridinium salts.

The acid salt is suitably an acid addition salt formed by a free acid. The free acid may be an inorganic or organic acid. Examples of suitable inorganic acids include, but are not limited to, hydrochloric acid, hydrobromic acid, sulfuric acid, sulfurous acid, phosphorous acid, diphosphoric acid, and nitric acid. Examples of suitable organic acids include, but are not limited to, citric acid, acetic acid, maleic acid, malic acid, fumaric acid, gluconic acid, methanesulfonic acid, benzenesulfonic acid, camphorsulfonic acid, oxalic acid, malonic acid, glutaric acid, glycolic acid, succinic acid, tartaric acid, 4-toluenesulfonic acid, galacturonic acid, embonic acid, glutamic acid, citric acid, aspartic acid, and stearic acid. However, the acid salt is intended to include those formed by various inorganic and organic acids commonly used in the art.

The muscarinic receptor antagonist is intended to include all salts, hydrates, solvates, derivatives, etc. that can be prepared by conventional methods, as well as pharmaceutically or sitologically acceptable salts. The addition salt may be prepared by a suitable method known in the art. Specifically, the addition salt is prepared by dissolving the muscarinic receptor antagonist in a water miscible organic solvent such as acetone, methanol, ethanol or acetonitrile, adding an excess of an organic base or an aqueous solution of an inorganic base to the solution, and precipitating or crystallizing the mixture. Alternatively, the mixture may be evaporated to remove the solvent or the excess base and dried. The precipitated salt may be optionally filtered under suction.

The pharmaceutical composition of the present invention may further include a suitable carrier, excipient or diluent known in the art. Examples of carriers, excipients, and diluents that can be used in the pharmaceutical composition include, but are not limited to, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, methyl hydroxybenzoate, propyl hydroxybenzoate, talc, magnesium stearate, and mineral oil.

The pharmaceutical composition of the present invention can be formulated into oral preparations such as powders, granules, tablets, capsules, suspensions, emulsions, syrups, and aerosols, and other preparations such as external preparations, suppositories, and sterile injectable solutions according to a conventional method suitable for each preparation. Specifically, the pharmaceutical composition may be formulated with diluents or excipients commonly used in the art, such as fillers, extenders, binders, wetting agents, disintegrating agents or surfactants. Solid preparations for oral administration include tablets, pills, powders, granules, and capsules. Such solid preparations may be prepared by mixing the pharmaceutical composition with at least one excipient, for example, starch, calcium carbonate, sucrose, lactose or gelatin. In addition to the simple excipients, lubricating agents such as magnesium stearate and talc may also be used. Liquid preparations for oral administration are suspensions, solutions for internal use, emulsions, and syrups. The liquid preparations may include various excipients, for example, wetting agents, sweeteners, aromatic substances, and preservatives, as well as simple diluents known in the art, such as water and liquid paraffin. Sterilized aqueous solutions, non-aqueous solvents, suspensions, emulsions, lyophilizates, and suppositories are included in preparations for parenteral administration. The non-aqueous solvents and the suspensions may be propylene glycol, polyethylene glycol, vegetable oils such as olive oil, and injectable esters such as ethyl oleate. Witepsol, Macrogol, Tween 61, cacao butter, laurin butter, and glycerogelatin may be used as bases of the suppositories.

The pharmaceutical composition of the present invention may be administered by any appropriate route of administration, preferably orally or parenterally. Examples of parenteral routes of administration include, but are not limited to, intravenous, intramuscular, intraarterial, intramedullary, intradural, intracardiac, transdermal, subcutaneous, intraperitoneal, intranasal, gastrointestinal, topical, sublingual, and intrarectal routes. As used herein, the term "parenteral" includes subcutaneous, intradermal, intravenous, intramuscular, intraarticular, intrasynovial, intrasternal, intradural, intraperitoneal, intralesional, and intracranial injectable preparations, and transrectal and transdermal preparations. The pharmaceutical composition of the present invention may also be administered in the form of a suppository for rectal administration.

The pharmaceutical composition of the present invention is preferably administered parenterally. Most preferably, the pharmaceutical composition of the present invention is administered in the form of an injectable preparation or a transrectal or transdermal preparation.

The pharmaceutical composition of the present invention may vary depending on a variety of factors, including activity of the particular compound, age, weight, sex, general health, diet, time and route of administration, excretion rate, combination with other drugs, and severity of the particular disease being treated. The dose of the pharmaceutical composition may vary depending on the general health and body weight of the patient, the severity of the disease, the drug formulation, and the route and time of administration. In consideration of these factors, those skilled in the art can determine an appropriate dose of the pharmaceutical composition. The pharmaceutical composition may be administered in a daily dose of 0.0001 to 50 mg/kg, 0.001 to 50 mg/kg, 0.01 to 50 mg/kg, 0.1 to 30 mg/kg, 1 to 30 mg/kg or 10 to 30 mg/kg. The pharmaceutical composition may be administered in single or divided doses per day. The pharmaceutical composition of the present invention may be formulated into pills, dragees, capsules, liquids, gels, syrups, slurries, and suspensions. The pharmaceutical composition of the present invention may be used either alone or in combination with surgery or other therapeutic agents for the prevention or treatment of nicotine withdrawal.

The present invention will be explained in more detail with reference to the following examples. However, these examples are not to be construed as limiting or restricting the invention, which is defined by the appended claims.

The experimental results of the following examples, including comparative examples, are merely representative and the effects of the exemplary embodiments of the present invention that are not explicitly presented hereinafter can be specifically found in the corresponding sections.

### Experimental Example 1. Determination of efficacy of muscarinic receptor antagonist in preventing or treating nicotine withdrawal symptoms - 1

### Experimental animals

Experimental animals, aged ≥ 7 weeks, were purchased from the Laboratory Animal Resources Center, Korea Institute of Science and Technology (KIST). All animal procedures were performed with the approval of the Animal Care and Use Committee of KIST. The experimental animals were acclimatized to the laboratory environment for at least one week prior to starting the experiment and behavioral abnormalities of the animals were observed. Animals with abnormalities were excluded from this experiment. All experimental animals were housed with a 12-h light/dark cycle and had *ad libitum* access to water and food in cages under controlled temperature and humidity.

### Sample administration and sampling

For sample administration, the mice were randomly divided into 3 groups, 10 animals per group. For a control, nicotine (0.15 mg/10 ml/kg) was injected intraperitoneally once daily for 3 days (I.P. injection). On day 5 (D5), saline was injected intraperitoneally. 30 min later, mecamylamine (MEC, 3.0 mg/10 ml/kg) was administered. For Proc3, nicotine (0.15 mg/10 ml/kg) was injected intraperitoneally once daily for 3 days (I.P. injection). On day 5 (D5), procyclidine (3.0 mg/10 ml/kg) was injected intraperitoneally. 30 min later, mecamylamine (MEC, 3.0 mg/10 ml/kg) was administered. For Proc10, nicotine (0.15 mg/10 ml/kg) was injected intraperitoneally once daily for 3 days (I.P. injection). On day 5 (D5), procyclidine (10.0 mg/10 ml/kg) was injected intraperitoneally. 30 min later, mecamylamine (MEC, 3.0 mg/10 ml/kg) was administered. These groups were subjected to an open field test immediately after mecamylamine administration. Mecamylamine (MEC) is a drug that induces nicotine withdrawal.

### 3 groups of animal models

Group 1 (control): Nicotine (0.15 mg/10 ml/kg) was injected intraperitoneally once daily for 3 days. On day 5 (D5), saline (10 ml/kg) was injected intraperitoneally. 30 min later, mecamylamine (MEC, 3.0 mg/10 ml/kg) was administered intraperitoneally.

Group 2 (Proc3): Nicotine (0.15 mg/10 ml/kg) was injected intraperitoneally once daily for 3 days. On day 5 (D5), procyclidine (3.0 mg/10 ml/kg) was injected intraperitoneally. 30 min later, mecamylamine (MEC, 3.0 mg/10 ml/kg) was administered intraperitoneally.

Group 3 (Proc10): Nicotine (0.15 mg/10 ml/kg) was injected intraperitoneally once daily for 3 days. On day 5 (D5), procyclidine (10.0 mg/10 ml/kg) was injected intraperitoneally. 30 min later, mecamylamine (MEC, 3.0 mg/10 ml/kg) was administered intraperitoneally.

### Open field test

Experimental animals were allowed to freely move in an open behavior observation box (40×40×40 cm). The moving distances and activity times of the freely moving animals for 30 min were measured using an animal behavior observation system (EthoVision system, Noldus IT b.v., Netherlands).

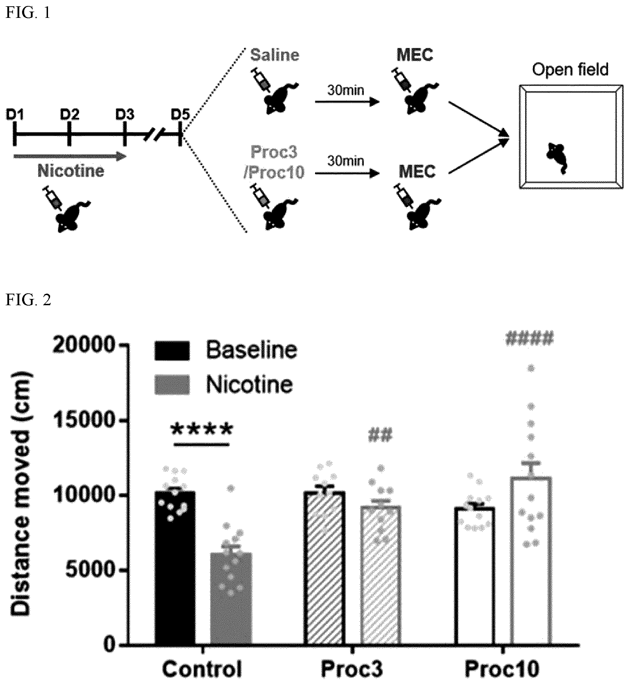

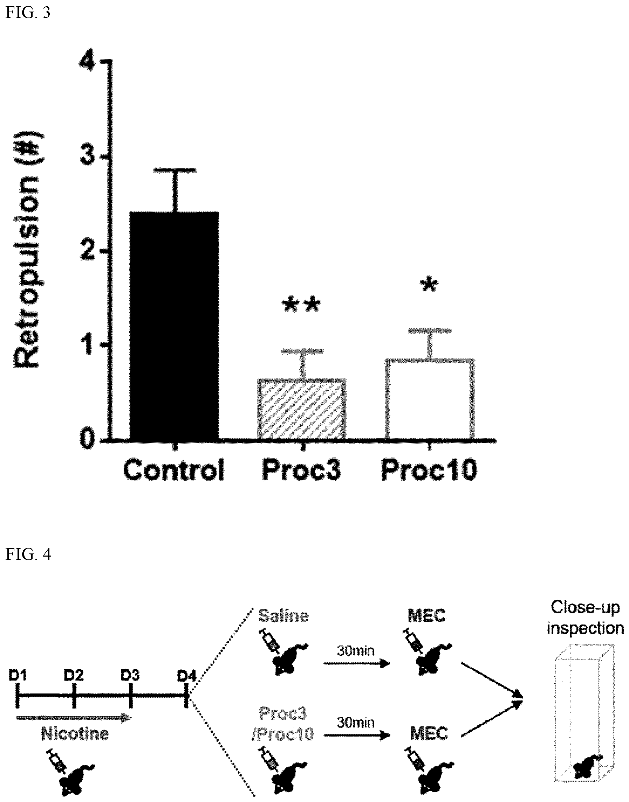

When an experimental animal is for the first time exposed to a new open place, its expressed exploratory behaviors have value as indicators of innate behavioral patterns because there are no memories and previous memories or episodes related to learning. The moving distance of each experimental animal in the open field was analyzed based on the observation of the exploratory behaviors for 10 min. The results are shown in Fig. 2. The degree of retropulsion of each experimental animal was analyzed based on the observation of the exploratory behaviors for 10 min. The results are shown in Fig. 3. Retropulsion refers to an abnormal backward falling behavior.

### Statistical analysis

All test results were expressed as mean ± SE. Statistical analysis was performed using two-way ANOVA and Bonferroni post-hoc tests. A value of P < 0.05 was considered significantly different compared to each experimental group.

Fig. 1 is an open field test design for determining the efficacy of the muscarinic receptor antagonist in preventing or treating nicotine withdrawal symptoms, Fig. 2 shows the moving distances of mice analyzed in the open field test for Groups 1 to 3, and Fig. 3 shows the degrees of retropulsion analyzed in the open field test for Groups 1 to 3. In Fig. 2, the baseline indicates the moving distance of mice in each group on day 0 before nicotine treatment.

As shown in Fig. 2, when MEC was administered to Group 1 (control) to cause nicotine withdrawal, the overall activity of mice was significantly suppressed more than twice by a factor of at least 2 (Control-Baseline vs. Control-Nicotine, ****p < 0.0001). In contrast, the decreased activities due to nicotine withdrawal in Group 2 (Proc3) and 3 (Proc10) were restored to normal levels (Control-Nicotine vs. Proc3-Nicotine, ##p < 0.01; Control-Nicotine vs. Proc10-Nicotine, ####p < 0.0001).

As shown in Fig. 3, no retropulsion should be found in the normal group but a significant increase in retropulsion was observed in Group 1 (control) where nicotine withdrawal was induced. That is, nicotine withdrawal induced retropulsion in mice.

In contrast, the administration of procyclidine (Proc3, Proc10), a muscarinic receptor antagonist, inhibited nicotine withdrawal-induced retropulsion by a factor of at least 2 regardless of the procyclidine concentration (Control vs. Proc3, **p < 0.01; Control vs. Proc10, *p < 0.05).

The increased concentration of procyclidine (Proc), a muscarinic receptor antagonist, effectively inhibited nicotine withdrawal symptoms by at least 1.5 times. These results concluded that the muscarinic receptor antagonist ameliorates, prevents or treats physical symptoms caused by nicotine withdrawal. That is, muscarinic receptor antagonists, including procyclidine (proc), are demonstrated to have prophylactic or therapeutic effects on nicotine withdrawal symptoms.

### Experimental Example 1. Determination of efficacy of muscarinic receptor antagonist in preventing or treating nicotine withdrawal symptoms - 2

### Experimental animals

Experimental animals, aged ≥ 7 weeks, were purchased from the Laboratory Animal Resources Center, Korea Institute of Science and Technology (KIST). All animal procedures were performed with the approval of the Animal Care and Use Committee of KIST. The experimental animals were acclimatized to the laboratory environment for at least one week prior to starting the experiment and behavioral abnormalities of the animals were observed. Animals with abnormalities were excluded from this experiment. All experimental animals were housed with a 12-h light/dark cycle and had *ad libitum* access to water and food in cages under controlled temperature and humidity.

### Sample administration and sampling

For sample administration, the mice were randomly divided into 3 groups, 10 animals per group. For a control, nicotine (0.15 mg/10 ml/kg) was injected intraperitoneally once daily for 3 days (I.P. injection). On day 4 (D4), saline was injected intraperitoneally. 30 min later, mecamylamine (MEC, 3.0 mg/10 ml/kg) was administered. For Proc3, nicotine (0.15 mg/10 ml/kg) was injected intraperitoneally once daily for 3 days (I.P. injection). On day 4 (D4), procyclidine (3.0 mg/10 ml/kg) was injected intraperitoneally. 30 min later, mecamylamine (MEC, 3.0 mg/10 ml/kg) was administered. For Proc10, nicotine (0.15 mg/10 ml/kg) was injected intraperitoneally once daily for 3 days (I.P. injection). On day 4 (D4), procyclidine (10.0 mg/10 ml/kg) was injected intraperitoneally. 30 min later, mecamylamine (MEC, 3.0 mg/10 ml/kg) was administered. These groups were subjected to a paw tremor test immediately after mecamylamine administration. Mecamylamine (MEC) is a drug that induces nicotine withdrawal.

### 3 groups of animal models

Group 1 (control): Nicotine (0.15 mg/10 ml/kg) was injected intraperitoneally once daily for 3 days. On day 4 (D4), saline (10 ml/kg) was injected intraperitoneally. 30 min later, mecamylamine (MEC, 3.0 mg/10 ml/kg) was administered intraperitoneally.

Group 2 (Proc3): Nicotine (0.15 mg/10 ml/kg) was injected intraperitoneally once daily for 3 days. On day 4 (D4), procyclidine (3.0 mg/10 ml/kg) was injected intraperitoneally. 30 min later, mecamylamine (MEC, 3.0 mg/10 ml/kg) was administered intraperitoneally.

Group 3 (Proc10): Nicotine (0.15 mg/10 ml/kg) was injected intraperitoneally once daily for 3 days. On day 4 (D4), procyclidine (10.0 mg/10 ml/kg) was injected intraperitoneally. 30 min later, mecamylamine (MEC, 3.0 mg/10 ml/kg) was administered intraperitoneally.

### Paw tremor test

Experimental animals were allowed to freely move in a transparent acrylic rectangular box (7×7×30 cm). The movements of the freely moving animals for 10 min were closely measured using an animal behavior observation system (EthoVision system, Noldus IT b.v., Netherlands).

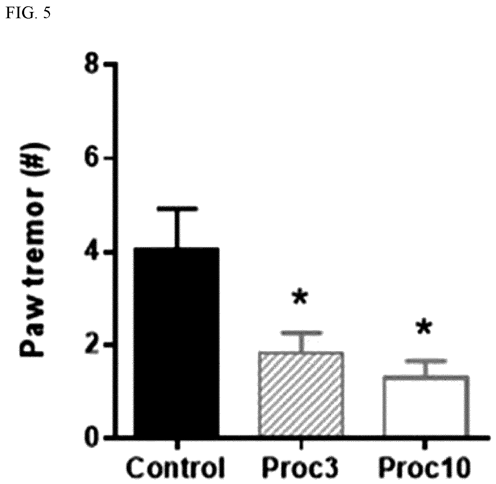

Since nicotine withdrawal induced paw tremors in experimental animals, the animals were confined in a transparent cylindrical box and their paw tremors were measured to analyze the physical dependence on nicotine. The degrees of paw tremors for 10 min were analyzed. The results are shown in Fig. 5.

### Statistical analysis

All test results were expressed as mean ± SE. Statistical analysis was performed using two-way ANOVA and Bonferroni post-hoc tests. A value of P < 0.05 was considered significantly different compared to Group 1 (control).

Fig. 4 is a paw tremor test design for determining the efficacy of the muscarinic receptor antagonist in preventing or treating nicotine withdrawal symptoms and Fig. 5 shows the degrees of paw tremors analyzed for Groups 1 to 3.

As shown in Fig. 5, no paw tremor behaviors should be found in the normal group but a significant increase in paw tremor was observed in Group 1 (control) where nicotine withdrawal was induced. That is, nicotine withdrawal induced paw tremors as physical symptoms in mice.

In contrast, the administration of procyclidine (Proc3, Proc10), a muscarinic receptor antagonist, suppressed paw tremors by a factor of at least 2 regardless of the procyclidine concentration (Control vs. Proc3, *p < 0.05; Control vs. Proc10, *p < 0.05).

The increased concentration of procyclidine (Proc), a muscarinic receptor antagonist, effectively inhibited nicotine withdrawal symptoms by at least 1.5 times. These results concluded that the muscarinic receptor antagonist has a prophylactic or therapeutic activity for physical symptoms caused by nicotine withdrawal.

## Claims

1. A pharmaceutical composition for use in preventing or treating nicotine withdrawal symptoms comprising a muscarinic receptor antagonist as an active ingredient, wherein the muscarinic receptor antagonist is selected from the group consisting of procyclidine, dexetimide, methixene, propiverine, benztropine, propenamine, propantheline, quinidine, glycopyrronium, orphenadrine, chlorphenoxamine, butylscopolamine, revefenacin, otilonium, emepronium, bornaprine, propiomazine, promethazine, pipecuronium, pancuronium, terfenadine, chlorprothixene, brompheniramine, mivacurium, isopropamide, tramadol, umeclidinium, hexocyclium, dosulepin, imidafenacin, pizotifen, thonzylamine, gallamine triethiodide, rocuronium, doxacurium, metocurine, dimetindene, diphenhydramine, rapacuronium, lamotrigine, camylofin, difemerine, dihexyverine, mebeverine, piperidolate, rociverine, trimebutine, emetonium iodide, benzilone, bevonium, diphemanil, fenpiverinium, penthienate, pipenzolate, poldine, prifinium, tiemonium iodide, timepidium, phenglutarimide, oxitropium, tropatepine, mazaticol, etybenzatropine, etanautine, batefenterol, and mixtures thereof, wherein the nicotine withdrawal symptoms are retropulsion and/or hand tremor.

2. The pharmaceutical composition for use according to claim 1, wherein muscarinic receptor antagonist is an antagonist of muscarinic acetylcholine receptor M1, muscarinic acetylcholine receptor M2, muscarinic acetylcholine receptor M3, muscarinic acetylcholine receptor M4, muscarinic acetylcholine receptor M5, and combinations thereof.

3. The pharmaceutical composition for use according to claim 1 or 2, wherein the pharmaceutical composition is formulated into an oral preparation and wherein the oral preparation being selected from the group consisting of powders, granules, tablets, capsules, suspensions, emulsions, syrups, and aerosols, and other preparations such as external preparations, suppositories, and sterile injectable solutions.

4. The pharmaceutical composition for use according to claim 1 or 2, wherein the pharmaceutical composition is formulated into a parenteral preparation and wherein the parenteral preparation is an injectable, transrectal or transdermal preparation.

## Patentansprüche

1. Pharmazeutische Zusammensetzung zur Verwendung bei der Vorbeugung oder Behandlung von Nikotinentzugserscheinungen, umfassend einen Muskarinrezeptorantagonisten als Wirkstoff, wobei der Muskarinrezeptorantagonist ausgewählt ist aus der Gruppe bestehend aus Procyclidin, Dexetimid, Methixen, Propiverin, Benztropin, Propenamin, Propanthelin, Chinidin, Glycopyrronium, Orphenadrin, Chlorphenoxamin, Butylscopolamin, Revefenacin, Otilonium, Emepronium, Bornaprin, Propiomazin, Promethazin, Pipecuronium, Pancuronium, Terfenadin, Chlorprothixen, Brompheniramin, Mivacurium, Isopropamid, Tramadol, Umeclidinium, Hexocyclium, Dosulepin, Imidafenacin, Pizotifen, Thonzylamin, Gallamintriethiodid, Rocuronium, Doxacurium, Metocurine, Dimetindene, Diphenhydramin, Rapacuronium, Lamotrigin, Camylofin, Difemerin, Dihexyverin, Mebeverin, Piperidolat, Rociverin, Trimebutin, Emetoniumiodid, Benzilon, Bevonium, Diphemanil, Fenpiverinium, Penthienat, Pipenzolat, Poldin, Prifinium, Tiemoniumiodid, Timepidium, Phenglutarimid, Oxitropium, Tropatepin, Mazaticol, Etybenzatropin, Etanautin, Batefenterol und Gemischen davon, wobei die Nikotinentzugserscheinungen Retropulsion und/oder Handzittern sind.

2. Pharmazeutische Zusammensetzung zur Verwendung nach Anspruch 1, wobei der Muskarinrezeptorantagonist ein Antagonist des muskarinischen Acetylcholinrezeptors M1, des muskarinischen Acetylcholinrezeptors M2, des muskarinischen Acetylcholinrezeptors M3, des muskarinischen Acetylcholinrezeptors M4, des muskarinischen Acetylcholinrezeptors M5 und Kombinationen davon ist.

3. Pharmazeutische Zusammensetzung zur Verwendung nach Anspruch 1 oder 2, wobei die pharmazeutische Zusammensetzung zu einer oralen Zubereitung formuliert ist und wobei die orale Zubereitung ausgewählt ist aus der Gruppe bestehend aus Pulvern, Granulaten, Tabletten, Kapseln, Suspensionen, Emulsionen, Sirupen und Aerosolen sowie anderen Zubereitungen wie äußerlichen Zubereitungen, Zäpfchen und sterilen injizierbaren Lösungen.

4. Pharmazeutische Zusammensetzung zur Verwendung nach Anspruch 1 oder 2, wobei die pharmazeutische Zusammensetzung zu einer parenteralen Zubereitung formuliert ist und wobei die parenterale Zubereitung eine injizierbare, transrektale oder transdermale Zubereitung ist.

## Revendications

1. Composition pharmaceutique destinée à être utilisée dans la prévention ou le traitement de symptômes de sevrage de la nicotine, la composition pharmaceutique comprenant un antagoniste des récepteurs muscariniques comme ingrédient actif, dans laquelle l'antagoniste des récepteurs muscariniques est choisi dans le groupe consistant en procyclidine, dexétimide, méthixène, propivérine, benztropine, propé-namine, propanthéline, quinidine, glycopyrronium, orphénadrine, chlorphénoxa-mine, butylscopolamine, révéfénacine, otilonium, émepronium, bornaprine, propiomazine, prométhazine, pipecuronium, pancuronium, terfénadine, chlor-prothixène, bromphéniramine, mivacurium, isopropamide, tramadol, uméclidinium, hexocyclium, dosulépine, imidafénacine, pizotifène, thonzylamine, triéthiodure de gallamine, rocuronium, doxacurium, métocurine, dimétindène, diphénhydramine, rapacuronium, lamotrigine, camylophine, difémérine, dihexyverine, mébévérine, pipéridolate, rociverine, trimébutine, iodure d'émétonium, benzilone, bévonium, diphémanil, fenpivérinium, penthiénate, pipenzolate, poldine, prifinium, iodure de tiémonium, timépidium, phenglutarimide, oxitropium, tropatépine, mazaticol, étybenzatropine, éthanautine, batéfentérol, et leurs mélanges, dans laquelle les symptômes de sevrage de la nicotine sont la rétropulsion et/ou des tremblements des mains.

2. Composition pharmaceutique destinée à être utilisée selon la revendication 1, dans laquelle l'antagoniste des récepteurs muscariniques est un antagoniste des récepteurs muscariniques de l'acétylcholine Ml, des récepteurs muscariniques de l'acétylcholine M2, des récepteurs muscariniques de l'acétylcholine M3, des récepteurs muscariniques de l'acétylcholine M4, des récepteurs muscariniques de l'acétylcholine M5 et des combinaisons de ceux-ci.

3. Composition pharmaceutique destinée à être utilisée selon la revendication 1 ou 2, dans laquelle la composition pharmaceutique est formulée en une préparation orale et dans laquelle la préparation orale est choisie dans le groupe consistant en poudres, granulés, comprimés, capsules, suspensions, émulsions, sirops et aérosols, et en autres préparations telles que des préparations externes, des suppositoires et des solutions injectables stériles.

4. Composition pharmaceutique destinée à être utilisée selon la revendication 1 ou 2, dans laquelle la composition pharmaceutique est formulée en une préparation parentérale et dans laquelle la préparation parentérale est une préparation injectable, transrectale ou transdermique.
